# EUROPEAN PATENT APPLICATION

(11) **EP 3 459 462 A1**
(43) Date of publication of application: **27.03.2019**
(21) Application number: 17193060.5
(22) Date of filing: 26.09.2017
(51) Int. Cl.: A61B 6/00, G05D 1/00

(54) **MOBILE X-RAY IMAGING SYSTEM**

(71) Applicant: Koninklijke Philips N.V., 5656 AE Eindhoven (NL)
(72) Inventor: STEADMAN BOOKER, Roger, 5656 AE Eindhoven (NL); DOKANIA, Anand Kumar, 5656 AE Eindhoven (NL); VON BERG, Jens, 5656 AE Eindhoven (NL)
(74) Representative: de Haan, Poul Erik

(57) **Abstract**

An X-ray imaging system (10) for medical imaging is provided. The imaging system (10) comprises an X-ray source (12) for emitting X-rays, an X-ray detector (14) for detecting X-rays, at least one unmanned aircraft (16), and at least one controller (18) for controlling at least one of the X-ray source (12), the X-ray detector (14), and the at least one unmanned aircraft (16). The X-ray source (12) or the X-ray detector (14) is arranged on the at least one unmanned aircraft (16), wherein the X-ray imaging system (10) is configured to capture an X-ray image of an object (15) arranged between the X-ray source (12) and the X-ray detector (14).

## Description

### FIELD OF THE INVENTION

Generally, the invention relates to the field of medical X-ray imaging. Particularly, the invention relates to an X-ray imaging system for medical imaging, to an X-ray arrangement and to a method for acquiring an X-ray image with such X-ray imaging system.

### BACKGROUND OF THE INVENTION

Recently, mobile X-ray imaging systems and/or devices have been developed, which may be lightweight and/or portable compared to conventional X-ray imaging systems e.g. used in hospitals or the like.

However, ensuring proper imaging conditions of the equipment for medical imaging, such as e.g. human medical X-ray examinations, usually requires a mechanical contraption where at least a part of the X-ray imaging system is affixed to. Further, e.g. when using handheld X-ray imaging devices an adequate field of view and/or an adequate source to detector distance for a given imaging task may only hardly be ensured. This may cause poor image quality and may require to repeat an acquisition several times, which may adversely affect a dose delivered to a patient during X-ray exposure.

### SUMMARY OF THE INVENTION

It would be advantageous to have an improved X-ray imaging system that is operable in a versatile manner and that provides improved X-ray image quality while reducing X-ray exposure and dose.

According to a first aspect of the invention, an X-ray imaging system for medical imaging is provided. The X-ray imaging system may particularly refer to a mobile and/or an ultra-mobile X-ray imaging system. Further, the X-ray imaging system may refer to a digital X-ray imaging system. The imaging system comprises an X-ray source for emitting X-rays and an X-ray detector for detecting X-rays. The X-ray imaging system further comprises at least one unmanned aircraft and at least one controller for controlling at least one of the X-ray source, the X-ray detector, the at least one unmanned aircraft and/or a movement of the at least one unmanned aircraft. The X-ray source or the X-ray detector is arranged on, mounted to, attached to, and/or fixed to the at least one unmanned aircraft, wherein the X-ray imaging system is configured to capture and/or acquire an X-ray image of an object arranged between the X-ray detector and the X-ray source.

Accordingly, the X-ray imaging system and/or the X-ray detector may be configured to capture and/or acquire a transmission X-ray image of the object. In other words, the X-ray imaging system and/or the X-ray detector may be configured to detect X-rays that have passed through the object. The object may generally refer to any object to be examined. By way of example the object may refer to a part of a patient, a body part of a patient, an entire patient and/or any other object.

The X-ray source may be any type of X-ray source configured to emit an arbitrary energy spectrum of X-rays. The X-ray source may comprise e.g. an X-ray tube for emitting an X-ray beam, such as e.g. a cone-beam.

The X-ray detector may particularly refer to a digital X-ray detector configured to capture a digital X-ray image. The X-ray detector may comprise one or more detecting elements and/or X-ray sensors. The X-ray detector may comprise e.g. an array of X-ray sensors arranged on a support, such as e.g. a TFT X-ray detector array. Further, the X-ray detector may comprise at least one converter for converting X-rays into visible light, such as e.g. a scintillator, which visible light may be detected with the one or more X-ray sensors.

The controller may refer to a control circuit, a control circuitry, a control module and/or a controller arrangement. The controller may be configured to at least partly control the unmanned aircraft, a movement of the unmanned aircraft, the X-ray source and/or the X-ray detector. Accordingly, the term "controlling the unmanned aircraft" and/or "configured to control the unmanned aircraft" may comprise controlling the movement of the unmanned aircraft. The controller may be configured to control the unmanned aircraft, a movement of the unmanned aircraft, the X-ray source and/or the X-ray detector based on remote control, e.g. based on receiving and/or processing a control signal from a control station and/or a control device, such as e.g. a computer, a notebook, a handheld device, a tablet PC, a smart phone, or the like. The controller may comprise at least one processor and/or a plurality of processors, wherein a single processor and/or a subset of the processors may refer to a sub-controller of the controller. Accordingly, the controller may comprise a plurality of sub-controllers, which may be arranged on different parts and/or components of the X-ray imaging system. The controller, at least one sub-controller and/or at least one processor may be arranged and/or located in the at least one unmanned aircraft, in a further unmanned aircraft of the X-ray imaging system, in the X-ray source and/or in the X-ray detector.

The X-ray imaging system may comprise at least one data storage configured to store and/or storing computer-executable instructions, which when executed by the controller, one or more of the sub-controllers, and/or one or more of the processors instruct the imaging system to carry out any of the functions described above and in the following with respect to the X-ray imaging system, with respect to a component and/or a part of the X-ray imaging system, and/or with respect to any other aspect of the invention, such as the X-ray imaging arrangement and the method. The data storage may comprise a plurality of data storage devices, e.g. arranged in different parts and/or components of the imaging system. Accordingly, at least one data storage device may be arranged in the unmanned aircraft, a further unmanned aircraft, the X-ray detector and/or the X-source.

The at least one unmanned aircraft may refer to an unmanned aerial vehicle, a drone, a quadrocopter, and/or any other type of mobile module configured to fly. Accordingly, the at least one aircraft may comprise at least one propelling unit, a drive and/or a motor enabling the at least one unmanned aircraft to fly and/or move three-dimensionally. The at least one unmanned aircraft may be at least in part remotely controlled. The at least one unmanned aircraft may comprise a communication interface, a communication module and/or a communication element to communicate, particularly biderectionally communicate, with another device, such as e.g. the control station and/or the control device.

Re-phrasing the first aspect of the invention, an X-ray imaging system is provided that comprises an X-ray source and an X-ray detector, wherein at least one of the X-ray source and the X-ray detector is mounted to an unmanned aircraft, and wherein the imaging system is configured to acquire a transmission X-ray image of an object arranged between the X-ray source and the X-ray detector. Arranging the X-ray detector or the X-ray source on the unmanned aircraft may advantageously increase a flexibility of the entire X-ray imaging system, particularly in terms of transportation, in terms of a positioning of the X-ray source and the X-ray detector with respect to each other and/or with respect to the object. An alignment of the X-ray detector and the X-ray source with respect to each other may advantageously be simplified, as the X-ray source or the X-ray detector mounted to the unmanned aircraft can be moved and positioned freely without any mechanical fixture and/or mechanical contraption. Also, this may further reduce a weight and/or mass of the entire imaging system. Apart from that, the X-ray imaging system may advantageously be transported at least partly by means of the unmanned aircraft. Particularly, the unmanned aircraft may be configured to transport both the X-ray source and the X-ray detector to an imaging site, where X-ray image acquisition is to be conducted and/or where the object is located. Accordingly, the X-ray imaging system may be partly or entirely mobile. The X-ray imaging system may advantageously be transported by means of the unmanned aircraft to any remote location, e.g. remote from any medical care, and/or to a difficult terrain. Accordingly, due to the mobility provided to the imaging system by the unmanned aircraft as well as the lightweight of the imaging system, access of the imaging system in less developed areas, in remote locations and/or in difficult terrains may be provided. Moreover, the X-ray imaging system may be configured to automatically acquire the X-ray image and/or it may be remotely controlled, such that no medical staff is required at the imaging site to perform the actual imaging task and/or to acquire the X-ray image. This may also increase a safety of the imaging system.

According to an embodiment of the invention, the unmanned aircraft may be configured to receive a control signal, e.g. from a control station and/or a control device. The control signal may e.g. comprise geographical coordinates of the imaging site, where the object to be examined is located. The control signal may also comprise data and/or information related to an imaging task specifying at least a part of the object to be examined with the X-ray imaging system. By way of example, the imaging task may refer to a part of a patient to be examined, such as e.g. a knee, a chest, an arm or any other body part. The unmanned aircraft may be configured, e.g. in response to receiving and/or processing the control signal, to navigate to the imaging site and/or to transport the X-ray source, the X-ray detector or both the X-ray source and the X-ray detector to the imaging site. The unmanned aircraft may also be configured to deploy the X-ray source, the X-ray detector or both the X-ray source and the X-ray detector at the imaging site.

In case the X-ray source is mounted to the unmanned aircraft, the unmanned aircraft and/or the controller may be configured to position and/or arrange the X-ray detector in a static location and/or a fixed position with respect to the object. For instance, the unmanned aircraft may be configured to deploy, particularly automatically deploy, the X-ray detector at the static location and/or the fixed position. Further, the controller may be configured to maneuver and/or position the unmanned aircraft and/or the X-ray source opposite to the X-ray detector, e.g. in a stationary position of the unmanned aircraft with respect to the object and/or the X-ray detector. Also, the controller may be configured to maneuver the unmanned aircraft, e.g. three-dimensionally, such that the X-ray source and the X-ray detector are aligned with respect to each other, particularly when the unmanned aircraft is in the stationary position. The controller may further be configured to trigger, particularly automatically trigger, X-ray image acquisition, when the X-ray detector and the X-ray source are aligned with respect to each other and/or when the X-ray source and/or the X-ray detector are positioned with respect to the object in the static position, respectively. Alternatively or additionally, X-ray image acquisition may be triggered in response to a control signal, e.g. received from a control device and/or a control station.

Vice versa, in case the X-ray detector is mounted to the unmanned aircraft, the unmanned aircraft and/or the controller may be configured to position and/or arrange the X-ray source in a static location and/or a fixed position with respect to the object. For instance, the unmanned aircraft may be configured to deploy, particularly automatically deploy, the X-ray source at the static location and/or the fixed position. Further, the controller may be configured to maneuver and/or position the unmanned aircraft and/or the X-ray detector opposite to the X-ray source, e.g. in a stationary position of the unmanned aircraft with respect to the object and/or the X-ray source. Also, the controller maybe configured to maneuver the unmanned aircraft, e.g. three-dimensionally, such that the X-ray detector and the X-ray source are aligned with respect to each other, particularly when the unmanned aircraft is in the stationary position. The controller may further be configured to trigger, particularly automatically trigger, X-ray image acquisition, when the X-ray detector and the X-ray source are aligned with respect to each other and/or when the X-ray source and/or the X-ray detector are positioned with respect to the object. Alternatively or additionally, X-ray image acquisition may be triggered in response to a control signal, e.g. received from a control device and/or a control station.

According to an embodiment of the invention, the X-ray imaging system is configured to capture an X-ray image of the object arranged between the X-ray detector and the X-ray source, when the at least one unmanned aircraft is in a stationary position, wherein the stationary position of the unmanned aircraft is a stationary flight position or a stationary position on ground. The controller may be configured to maneuver and/or navigate the unmanned aircraft to the stationary position. Therein, the stationary position of the unmanned aircraft may refer to a position, in which an orientation and/or a distance of the unmanned aircraft with respect to the object may be fixed. In the stationary position of the unmanned aircraft, the X-ray detector or the X-ray source mounted to the unmanned aircraft may be in a static and/or fixed position with respect to the object. Further, a relative orientation and/or a distance between the X-ray source and the X-ray detector, i.e. a source to detector distance, may be constant and/or fixed, when the unmanned aircraft is in the stationary position. By capturing the X-ray image in the stationary position, motion artefacts, e.g. due to a relative movement of the X-ray source and the X-ray detector and/or due to a movement of the X-ray source and/or the X-ray detector with respect to the object, may be avoided and/or minimized. Hence, an image quality of the X-ray image may be improved.

According to an embodiment of the invention, the X-ray imaging system comprises a further unmanned aircraft, wherein the X-ray source is arranged on, mounted to, attached to and/or fixed to the unmanned aircraft and the X-ray detector is arranged on, mounted to, attached to and/or fixed to the further unmanned aircraft. The imaging system is further configured to capture the X-ray image of the object, when the unmanned aircraft is in the stationary position and the further unmanned aircraft is in a further stationary position. Therein, the further stationary position of the further unmanned aircraft may be a stationary flight position or a stationary position on ground. E.g. depending on the imaging task and/or depending on a terrain at the imaging site one of the unmanned aircraft and the further unmanned aircraft may be in a stationary flight position, whereas the other one of the unmanned aircraft and the further unmanned aircraft may be in a stationary position on ground. Alternatively, both the unmanned aircraft and the further unmanned aircraft may be in a stationary position on ground or in a stationary flight position. When the unmanned aircraft is in the stationary position and the further unmanned aircraft is in the further stationary position, a relative orientation and/or a distance between the X-ray source and the X-ray detector may be fixed and/or constant. Also, a relative orientation and/or a distance of the unmanned aircraft and/or the further unmanned aircraft with respect to the object may be fixed and/or constant. This may allow to avoid motion artefacts and hence may allow to improve image quality of the X-ray image. Further, a dose delivered to the object may be reduced as only a single X-ray image acquisition may be required.

The controller may be configured to maneuver the unmanned aircraft to the stationary position and/or to control a movement of the unmanned aircraft to maintain the stationary position. Alternatively or additionally, the controller may be configured to maneuver the further aircraft to the further stationary position and/or to control a movement of the further unmanned aircraft to maintain the further stationary position.

The unmanned aircraft and the further unmanned aircraft each may comprise a communication element to communicate and/or exchange information with each other, with the controller, with a control device and/or with a control station. Such information can comprise information related to and/or indicative of a position, a movement, an acceleration, a speed, a direction of movement and/or a distance of a movement. The unmanned aircraft and/or the further unmanned aircraft may comprise one or more sensors, such as e.g. an accelerometer, a distance sensor, an optical sensor, a visual sensor, a camera, a radar distance sensor, a laser distance sensor, a gyroscope, a speed sensor, a wind speed sensor and/or any other type of sensor. The controller may be configured to process sensor signals of the one or more sensors of the unmanned aircraft and/or the further unmanned aircraft, and the controller may be configured to exchange information related to and/or indicative of any of the one or more sensors between the unmanned aircraft and the further unmanned aircraft via the communication elements of the unmanned aircraft and the further unmanned aircraft.

The controller may further be configured to coordinate a relative movement of the unmanned aircraft and the further unmanned aircraft, e.g. based on image data of images captured with at least one camera, based on information exchanged via the communication elements of the unmanned aircraft and/or the further unmanned aircraft and/or based on sensor signals from one or more of the sensors of the unmanned aircraft and/or of the further unmanned aircraft. For this purpose, each of the unmanned aircrafts may comprise a sub-controller of the controller. Alternatively or additionally, one of the unmanned aircraft and the further unmanned aircraft may be a master aircraft, whereas the other one of the unmanned aircraft and the further unmanned aircraft may be configured as a slave aircraft. The controller may be configured to maneuver the slave aircraft such that it follows a movement of the master aircraft and/or such that the relative orientation and/or the source to detector distance is kept constant, particularly during X-ray image acquisition. However, a movement of both the unmanned aircraft and the further unmanned aircraft may also be controlled independently from each other by the controller, by a control device and/or by a control station.

According to an embodiment of the invention, the at least one unmanned aircraft comprises an extendable support, on which the X-ray source or the X-ray detector is arranged, wherein the controller is configured to position the X-ray source or the X-ray detector by actuating the extendable support of the unmanned aircraft, such that the X-ray source and the X-ray detector are arranged opposite to each other. The controller maybe configured to actuate the extendable support in the stationary position of the unmanned aircraft. By way of example, the extendable support may comprise a tripod. One end of the extendable support may be fixed at the unmanned aircraft and an opposite end of the extendable support may be extended in direction of a surface of a ground, such that the unmanned aircraft may be lifted from ground by extending the extendable support. Alternatively, the X-ray source or the X-ray detector may be arranged and/or fixed at the opposite end of the extendable support, such that only the X-ray source and/or the X-ray detector may be moved by extending the extendable support. Both the unmanned aircraft and the further unmanned aircraft may comprise such an extendable support. By means of the extendable support the X-ray source and the X-ray detector may be positioned and/or aligned with respect to each other. Also, it may be ensured that the relative orientation and the source to detector distance is constant and/or fixed during X-ray image acquisition.

According to an embodiment of the invention, at least one of the X-ray source, the X-ray detector, the unmanned aircraft and/or the further unmanned aircraft is battery powered. Particularly, the entire imaging system may be battery powered. Battery powering at least a part of the imaging system or the entire imaging system may advantageously reduce an overall weight of the imaging system as well as increase movability, portability and/or flexibility of the imaging system.

According to an embodiment of the invention, the at least one controller is configured to determine and/or calculate a value of at least one acquisition parameter based on an imaging task, the imaging task specifying at least a part of the object to be examined with the X-ray imaging system, wherein the at least one controller is configured to automatically adjust the value of the at least one acquisition parameter by controlling at least one of the X-ray source, the X-ray detector, the at least one unmanned aircraft, and a movement of the at least one unmanned aircraft. By way of example, the imaging task may refer to a part of a patient to be examined, such as e.g. a knee, a chest, an arm, a thorax, a head or any other body part. The value of the at least one acquisition parameter may be automatically determined as well as automatically adjusted by the controller. Generally, this allows the imaging system to be at least partly automated. Also, there may be no need for any medical staff at the imaging site due to this automation of the imaging system.

Alternatively or additionally, the value of at least one of the acquisition parameters may be transmitted to the imaging system via a communication interface of the imaging system.

According to an embodiment of the invention, the at least one acquisition parameter is selected from the group consisting of a source to detector distance, an X-ray voltage, an X-ray current, a field of view, a magnification, and an acquisition time. By way of example, the imaging task, such as e.g. a chest X-ray, may be specified and transmitted e.g. from a control device and/or a control station to the imaging system by means of a control signal and/or a command signal. Based on the transmitted and/or specified imaging task, the imaging system and/or the controller may then automatically determine values of at least a part of the acquisition parameters, preferably of all of the acquisition parameters, such as e.g. an appropriate X-ray current, an appropriate X-ray voltage, an appropriate field of view, an appropriate magnification, an appropriate source to detector distance, an appropriate acquisition time instant and/or an appropriate acquisition time period. Alternatively or additionally, values of the acquisition parameters for each imaging task may be stored in a data storage of the imaging device, such as e.g. a look-up table. The controller may the automatically adjust one or more of the determined values, preferably the controller may adjust all of the determined values. This adjustment may comprise positioning the unmanned aircraft, the further unmanned aircraft, the X-ray source and/or the X-ray detector with respect to the object and/or with respect to each other. Particularly, the controller may maneuver the unmanned aircraft and/or the further unmanned aircraft such that a value of the source to detector distance as determined based on the imaging task may be maintained and/or kept constant during image acquisition.

According to an embodiment of the invention, the imaging system further comprises at least one communication interface and/or a communication interface arrangement, wherein the X-ray imaging system is configured to receive data related to an imaging task via the communication interface, the imaging task specifying at least a part of the object to be examined with the X-ray imaging system. The at least one communication interface may be arranged e.g. on the unmanned aircraft and/or on a further unmanned aircraft of the imaging system. The at least one communication interface may comprise one or more communication elements. By way of example, the unmanned aircraft, the further unmanned aircraft, the X-ray source, the X-ray detector, and/or the controller each may comprise a communication element of the communication interface and/or the communication interface arrangement. Accordingly, the communication interface and/or the communication elements may be configured to establish and/or provide a communication interface between at least a part of the unmanned aircraft, the further unmanned aircraft, the X-ray source, the X-ray detector, the controller, and/or a control device. The at least one communication interface and/or the communication elements may be configured for wireless communication. The imaging system may also comprise a plurality of different communication interfaces. By way of example, the at least one communication interface may be configured for a satellite communication enabling remote control of at least a part of the imaging system via satellite. Alternatively or additionally the communication interface may be configured for a small to medium distance communication and/or for Wireless LAN communication, Bluetooth communication, Infrared communication, and/or radio frequency communication.

According to an embodiment of the invention, the X-ray imaging system is configured to transmit and/or send data related to the X-ray image via the communication interface. By way of example the controller and/or the X-ray detector may be configured to broadcast the data related to the X-ray image and/or image information of the X-ray image to a control device, a control station and/or a local viewing device. Alternatively or additionally, the controller and/or the X-ray detector maybe configured to broadcast the data related to the X-ray image e.g. directly to a referred phyisician who might not necessarily be at the imaging site, for instance via a cloud environment and/or via satellite communication. Apart from that any data gathered and/or determined by any component of the imaging system, such as e.g. the determined values of the acquisition parameters, data indicative of and/or related to sensor signals of any sensor of the imaging system, particularly any sensor of the unmanned aircraft and/or the further unmanned aircraft, may be transmitted via the communication interface. Such data may, for instance, comprise data related to an accelerometer sensor of at least one of the unmanned aircraft and the further unmanned aircraft. Further, such data may be used for image reconstruction and/or image processing, e.g. at the control station.

According to an embodiment of the invention, the at least one unmanned aircraft comprises at least one camera, wherein the controller is configured to position, particularly to automatically position, the at least one unmanned aircraft with respect to the object based on image recognition using the at least one camera, such that at least a part of the object to be examined with the X-ray imaging system is arranged between the X-ray source and the X-ray detector. Generally, this allows to automatically position the unmanned aircraft as well as the X-ray source or the X-ray detector mounted to the unmanned aircraft with respect to the object.

By way of example, the controller may be configured to process image data of one or more images captured with the at least one camera. Alternatively or additionally, the controller may be configured to determine and/or identify a part of the object to be examined based on image recognition and/or based on processing image data of the one or more images captured by the camera. For instance, an imaging task may specify that a knee of a patient is to be examined. The at least one camera may capture one or more images of the patient and the controller may be configured to identify the knee of the patient in the one or more images captured with the camera based on processing the image data of the one or more images and based on image recognition. Based on the specified imaging task and/or based on the image data of the one or more images captured with the camera, the controller may determine a position, e.g. a stationary position, of the unmanned aircraft, of the X-ray source and/or the X-ray detector with respect to the object and/or patient. The controller may further be configured to maneuver and/or navigate the unmanned aircraft to the determined position and/or the determined stationary position. Further, the controller may be configured to control a movement of the unmanned aircraft to maintain the determined position and/or stationary position for acquisition of the X-ray image.

However, both the unmanned aircraft and the further unmanned aircraft each may comprise at least one camera, wherein the controller may be configured to process image data of an image captured with any of the cameras. Also, image data and/or images may be exchanged between the unmanned aircraft and the further unmanned aircraft, e.g. via one or more communication interfaces and/or one or more communication elements.

According to an embodiment of the invention, the imaging system comprises a further unmanned aircraft, wherein the X-ray source is arranged on the unmanned aircraft and the X-ray detector is arranged on the further unmanned aircraft. At least one of the unmanned aircraft and the further unmanned aircraft comprises at least one camera. However, the unmanned aircraft may comprise at least one camera and the further unmanned aircraft may comprise at least one further camera. The at least one controller is configured to align, particularly automatically align, the X-ray source with respect to the X-ray detector by positioning the unmanned aircraft and the further unmanned aircraft with respect to each other based on image recognition using the at least one camera. Accordingly, the X-ray detector and the X-ray source may be aligned with respect to each other in a fully automated manner based on image recognition. Therein, aligning the X-ray source and the X-ray detector may comprise maintaining a relative orientation and/or a source to detector distance of the X-ray detector and the X-ray source.

According to an embodiment of the invention, the at least one controller is configured to determine a current and/or actual relative orientation of the X-ray source and the X-ray detector based on image data of the at least one camera and/or based on processing the image data. Alternatively or additionally, the at least one controller is configured to determine a current and/or actual source to detector distance between the X-ray source and the X-ray detector based on the image data of the camera and/or based on processing the image data, as well as based on a reference size of the X-ray detector, and based on a reference image size of the camera. In other words, the controller may be configured to process image data of one or more images captured with the at least one camera and to determine the current relative orientation and/or the current source to detector distance by processing the image data, taking into account the reference size of the X-ray detector and the reference image size of the camera. The reference size of the X-ray detector and/or the reference image size may e.g. be stored in a data storage of the imaging system. By way of example, the controller may be configured to process the image data and determine a detector plane in the image data. If the detector plane comprises parallel edges and/or boundaries, then the X-ray source and the X-ray detector may be aligned and/or may be arranged parallel to each other. In contrast, if the detector plane comprises trapezoidal edges and/or boundaries, the unmanned aircraft and/or the further unmanned aircraft may be moved horizontally and/or vertically until the detector plane comprises parallel edges and/or boundaries. By knowing the reference image size and the reference size of the X-ray detector, the controller may further calculate and/or determine the current source to detector distance. Also, the controller may be configured to maneuver the unmanned aircraft and/or the further unmanned aircraft until the current source to detector distance matches e.g. a source to detector distance as determined by the controller based on the specified imaging task.

Alternatively or additionally, at least one of the unmanned aircraft and the further unmanned aircraft may perform a predefined movement and to capture one or more images with the camera during this movement. Based on this predefined movement and based on the image data of the captured images, the controller may be configured to calculate and/or determine the current relative orientation and/or the current source to detector distance based on triangulation. In this case, the reference size of the X-ray detector and/or the reference image size must not necessarily be known.

According to an embodiment of the invention, the at least one controller is configured to determine a current relative orientation of the X-ray source and the X-ray detector based on a sensor signal of at least one sensor of the imaging system. For instance, the unmanned aircraft and/or the further unmanned may carry and/or comprise the at least one sensor, which may refer to e.g. an accelerometer, a distance sensor, an optical sensor, a visual sensor, a radar distance sensor, a laser distance sensor, a gyroscope, a speed sensor, a wind speed sensor and/or any other type of sensor. Based on one or more sensor signals from one or more of the sensors, the controller may determine a movement of the unmanned aircraft and/or the further unmanned aircraft and compensate the movement by maneuvering the unmanned aircraft and/or the further unmanned aircraft, such that the current relative orientation and/or the current source to detector distance matches the relative orientation and/or the source to detector distance as determined by the controller based on the imaging task.

According to an embodiment of the invention, the controller is configured to maintain the current relative orientation and/or the current source to detector distance during X-ray image acquisition by maneuvering at least one of the unmanned aircraft and the further unmanned aircraft and by positioning the unmanned aircraft and the further unmanned aircraft with respect to each other. Accordingly, the controller may be configured to determine the current source to detector distance and/or the current relative orientation based on processing image data of the camera. The controller may be configured to compare the current relative orientation and/or the current source to detector distance with the source to detector distance and/or the relative orientation as determined based on the specified imaging task. Further, the controller may be configured to compensate any deviation of the current source to detector distance from the source to detector distance as determined based on the specified imaging task by maneuvering and/or moving at least one of the unmanned aircraft and the further unmanned aircraft. Likewise, the controller may be configured to compensate for any deviation of the current relative orientation from the relative orientation as determined based on the specified imaging task by maneuvering and/or moving at least one of the unmanned aircraft and the further unmanned aircraft. Alternatively or additionally, the controller may be configured to maintain and/or lock the current relative orientation and/or the current source to detector distance, when the current source to detector distance matches the source to detector distance as determined by the controller based on the imaging task and/or when the current relative orientation matches the relative orientation as determined by the controller based on the imaging task

According to an embodiment of the invention, the controller is configured to maneuver and/or move the unmanned aircraft and/or the further unmanned aircraft along a trajectory around the object, wherein the imaging system is configured to scan at least a part of the object based on capturing a plurality of X-ray images along the trajectory. In other words, the imaging system may be configured for X-ray scanning and/or for X-ray scanning applications, such as e.g. Tomo-sysnthesis acquisition. The unmanned aircraft and the further unmanned aircraft may be maneuvered such that the X-ray source and the X-ray detector are moved along a C-arm like trajectory around the object, e.g. while maintaining the relative orientation and/or the source to detector distance as determined based on the imaging task. As both the unmanned aircraft and the further unmanned aircraft can be moved without any mechanical fixture, the object may be scanned along an arbitrary trajectory without repositioning the object itself. Generally, this may increase flexibility of the imaging system. Further, the trajectory and/or trajectory data indicative of the trajectory may be stored in a data storage, and optionally the trajectory and/or the trajectory data may be used for image processing and/or image reconstruction of the X-ray images acquired.

According to an embodiment of the invention, the at least one unmanned aircraft comprises at least one camera configured to capture a plurality of images during X-ray image acquisition of the object, wherein the controller is configured to determine a movement of the object during X-ray image acquisition based on image data of the plurality of images captured with the at least one camera, and wherein the controller is configured to compensate and/or correct for the movement of the object during X-ray image acquisition based on image data of the plurality of images captured with the at least one camera during X-ray image acquisition. By way of example, a movement of a patient during image acquisition may be determined by the controller by processing the image data, identifying a profile and/or an edge of the object in the image data, and by determining a shift of the object in consecutively captured images. By knowing the shift of the object, this movement of the object may be compensated for in the X-ray image by the controller.

According to a second aspect of the invention, an X-ray imaging arrangement for medical X-ray imaging is provided. The imaging arrangement comprises an X-ray imaging system, as described above and in the following, and a control device for at least partly controlling the X-ray imaging system, particularly for controlling at least one unmanned aircraft, the X-ray source and/or the X-ray detector, based on remote control. The control device may be a portable control device. The control device may comprise e.g. a computer, a notebook, a handheld device, a tablet PC, a smart phone, or the like. The control device may be configured to send one or more control signals to the imaging system. Particularly, the control device may be configured for remote control of the unmanned aircraft.

According to a third aspect of the invention, a method for acquiring an X-ray image of an object with an X-ray imaging system comprising an X-ray source, an X-ray detector and at least one unmanned aircraft is provided, wherein the X-ray source or the X-ray detector is mounted to the unmanned aircraft. The method may be a method for operating the X-ray imaging system, as described above and in the following, and/or a method for operating the X-ray imaging arrangement, as described above and in the following.

The method comprises the steps of:
- positioning the at least one unmanned aircraft with respect to an object to be examined, such that the object is arranged between the X-ray source and the X-ray detector;
- aligning the X-ray source and the X-ray detector by maneuvering and/or positioning the at least one unmanned aircraft; and
- capturing an X-ray image of the object arranged between the X-ray source and the X-ray detector.

It is to be noted that any feature, function, element and/or step described above and in the following with respect to one of the X-ray imaging system, the X-ray imaging arrangement and/or the method may be a feature, function, element and/or step of any other of the X-ray imaging system, the X-ray imaging arrangement and/or the method.

Also, it is to be noted that according to a further aspect, a computer program element may be provided, which, when executed on a controller and/or a processor of the imaging system, instructs the imaging system to carry out the steps of the method.

According to yet a further aspect, a computer readable medium may be provided, on which a computer program element may be stored, which, when executed on a controller and/or a processor of the imaging system, instructs the imaging system to carry out the steps of the method.

According to yet a further aspect, a use of at least one unmanned aircraft for medical X-ray imaging in an X-ray imaging system may be provided. Therein, an X-ray source and/or an X-ray detector may be mounted to the at least one unmanned aircraft.

These and other aspects of the invention will be apparent from and elucidated with reference to the embodiments described hereinafter.

### BRIEF DESCRIPTION OF THE DRAWINGS

The subject matter of the present disclosure will be explained in more detail in the following with reference to exemplary embodiments, which are illustrated in the attached figures.
Fig. 1 shows schematically an X-ray imaging arrangement according to an exemplary embodiment.
Fig. 2 shows schematically an X-ray imaging system according to an exemplary embodiment.
Fig. 3 shows schematically an X-ray imaging system according to a further exemplary embodiment.
Fig. 4 shows a flow chart illustrating steps of a method for acquiring an X-ray image according to an embodiment.

In principle, identical or similar parts are provided with identical reference numerals.

### DETAILED DESCRIPTION OF EMBODIMENTS

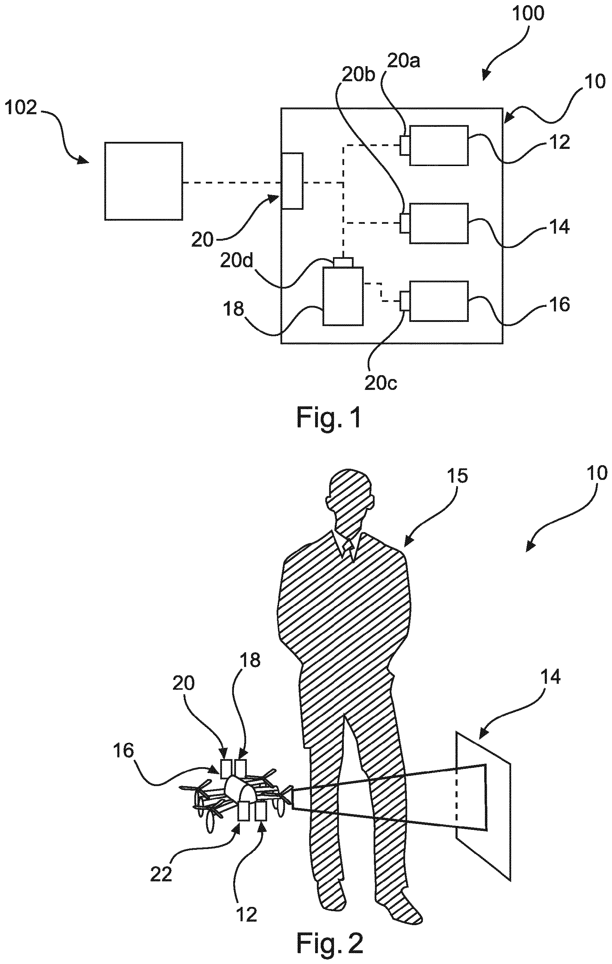

Fig. 1 shows schematically an X-ray imaging arrangement 100 according to an exemplary embodiment.

The X-ray imaging arrangement 100 comprises an X-ray imaging system 10 and a control device 102 for at least partly controlling the X-ray imaging system 10 based on and/or via remote control. The control device 102 may be located e.g. in a control station remote from a location of the imaging system 10. By means of the control device 102 e.g. a physician or any other medical staff can perform X-ray image acquisition via remotely controlling the imaging system 10.

As will be explained in more detail in subsequent figures, the X-ray imaging system comprises an X-ray source 12, an X-ray detector 14, at least one unmanned aircraft 16, and at least one controller 18.

Therein, one of the X-ray source 12 or the X-ray detector 14 is arranged on and/or mounted to the unmanned aircraft 16, wherein the X-ray imaging system 10 and/or the controller is configured to capture and/or acquire an X-ray image of an object 15 (see e.g. Figure 2) arranged between the X-ray source 12 and the X-ray detector 14.

Further, the imaging system 10 comprises at least one communication interface 20 and/or a communication interface arrangement 20 for communicating and/or for exchanging data with the control device 102. The communication interface 20 may particularly be configured for wireless communication between the control device 102 and the imaging system 10. The communication interface 20 maybe configured for communicating with the control device 102 via various communication channels. For instance, the communication interface 20 may be configured for Bluetooth communication, for radio frequency communication and/or for satellite communication.

The communication interface 20 may further be configured for wireless communication between any component of the imaging system 10, such as e.g. a communication between the unmanned aircraft 16 and the X-ray source 12, the X-ray detector 14, and/or the controller 18. For this purpose, the communication interface 20 may comprise a plurality of communication elements 20a-20d and/or communication modules 20a-20d. For instance, a communication element 20a may be arranged on the X-ray source 12, a communication element 20b may be arranged on the X-ray detector 14, a communication element 20c maybe arranged on the unmanned aircraft, and a communication element 20d may be arranged on and/or coupled to the controller. Via the communication elements 20a-20d, each of the unmanned aircraft 16, the controller 18, the X-ray source 12 and/or the X-ray detector 14 may communicate and/or exchange data with any other component of the imaging system, i.e. with the unmanned aircraft 16, the controller 18, the X-ray source 12 and/or the X-ray detector 14. Each of the communication elements 20a-20d or a part thereof may be configured for wireless communication, such as Bluetooth communication, radio frequency communication, infrared communication Wireless LAN communication and/or satellite communication.

Accordingly, by the communication interface 20 and/or the communication elements 20a-20d a communication network may be established and/or provided between the X-ray source 12, the X-ray detector 14, the unmanned aircraft 16, the controller 18 and/or the control device 102.

By means of the unmanned aircraft 16 both the X-ray source 12 and the X-ray detector 14 may be transported to an imaging site, where the X-ray image is to be acquired and/or where the object 15 to be examined is located. Accordingly, a self-delivery system may be provided by the at least one unmanned aircraft 16 and/or the imaging system 10. However, the unmanned aircraft 16, the X-ray source 12, the X-ray detector 16 and/or the controller 18 may be transported to the imaging site and/or close to the imaging site by any other kind of vehicle, e.g. an unmanned vehicle, such that longer distances may be covered and the load may be maximized by reducing the requirements for flight autonomy.

By way of example, a control signal maybe sent from the control device 102 to the imaging system 10, wherein the control signal may comprise geographic coordinates of the imaging site. The control signal may be processed by the controller 18 and the controller 18 may maneuver and/or navigate the unmanned aircraft 16, e.g. carrying the X-ray source 12 and/or the X-ray detector 14, to the imaging site.

Further, the controller 18 may be configured to instruct the unmanned aircraft 16 to deploy at least one of the X-ray source 12 and the X-ray detector 14 at the imaging site, whereas the other one of the X-ray source 12 and the X-ray detector is mounted to the unmanned aircraft 16.

It is to be noted that the controller 18 may be partly or fully integrated in the unmanned aircraft 16. However, the controller 18 may also comprise a plurality of sub-controllers, such as e.g. one or more processors, which may be arranged on different components of the imaging system 10. For instance, the unmanned aircraft 16 may comprise a sub-controller, the X-ray source may comprise a further sub-controller, and the X-ray detector may comprise a further sub-controller. Via the communication interface 20 and/or via the communication elements 20a-20d any commands, information and/or data may be exchanged between the various sub-controllers.

Fig. 2 shows schematically an X-ray imaging system 10 according to an exemplary embodiment. If not stated otherwise, the imaging system 10 of Fig. 2 comprises the same elements, features and/or functions as the imaging system 10 described with reference to Fig. 1.

In the embodiment depicted in Figure 2, the imaging system 10 comprises one unmanned aircraft 16 carrying the X-ray source 12, and the controller 18 is exemplary integrated into the unmanned aircraft 16. It is to be noted, however, that alternatively the X-ray detector 14 may be arranged and/or mounted to the unmanned aircraft 16.

The unmanned aircraft 16 may, e.g. based on a control signal received from the control device 102, transport the X-ray detector 14 to the imaging site and deploy the X-ray detector 14 at a static and/or fixed position with respect to the object 15, which is exemplary a patient 15 in the embodiment of Fig. 2. Therein, the X-ray detector 14 may be fixed at some mechanical fixture and/or contraption at the imaging site. In case the X-ray detector 14 is mounted to the unmanned aircraft 16, the X-ray source 12 may be transported to and deployed at the imaging site.

The unmanned aircraft 16 may further comprise at least one camera 22 for capturing optical images. Image data of the images captured with the camera 22 maybe processed by the controller 18.

Further, an imaging task may be transmitted to and/or selected by the controller 18, wherein the imaging task specifies a part of the object 15 and/or patient 15 to be examined, such as e.g. a knee, a lower leg, a leg, a thorax, a head, a chest, an arm or any other body part. For instance, the patient 15 could point to a region of interest, wherein the controller 18 may be configured to determine the region of interest based on image recognition using the camera 22 and/or based on image processing of image data of the images captured with the camera 22.

Alternatively or additionally, the imaging task may e.g. be selected on the control device 102 via an application, and a control signal containing data indicative of and/or related to the imaging task may be transmitted to the imaging system 10 via the communication interface 20.

Further, the controller 18 is configured to determine a value of at least one acquisition parameter based on the imaging task, wherein the at least one acquisition parameter is selected from the group consisting of a source to detector distance, an X-ray voltage, an X-ray current, a field of view, a magnification, and an acquisition time. Based on the imaging task, the controller 18 may calculate one or more values of one or more acquisition parameters. Alternatively or additionally, the values of the acquisition parameters may be stored in a storage device and/or a data storage of the imaging system 10, and the controller 18 may select the values based on the imaging task. For instance, a set of values of the acquisition parameters may be stored in the storage device and/or the data storage for each imaging task, and the controller 18 may select the set of values suitable for the imaging task currently specified, e.g. in the control signal of the control device 102.

Further, the controller 18 is configured to automatically adjust the value of the at least one acquisition parameter by controlling at least one of the X-ray source 12, the X-ray detector 14, the unmanned aircraft 16 and/or a movement of the unmanned aircraft 16. Specifically, the X-ray voltage, the X-ray current, the field of view, and/or the magnification may automatically be adjusted by controlling the X-ray source 12, the X-ray detector 14 and the unmanned aircraft 16 with the controller 18. Further, the controller 18 may maneuver and/or position the unmanned aircraft 16 such that an actual and/or a current source to detector distance matches the source to detector distance as determined based on the imaging task. Therein, the current source to detector distance may be determined by the controller 18 based on processing image data of one or more images captured with the camera 22, based on a reference size of the X-ray detector 14 and/or based on a reference image size of the camera 22. The controller 18 may compare the current source to detector distance with the source to detector distance as determined based on the imaging task, and the controller 18 may maneuver and/or position the unmanned aircraft 16 such that the current source to detector distance matches the source to detector distance as determined based on the imaging task.

The controller 18 may also be configured to determine a stationary position of the unmanned aircraft 16 with respect to the patient 15, in which stationary position the current source to detector distance may match the source to detector distance as determined based on the imaging task. The controller 18 may then navigate the unmanned aircraft 18 to the stationary position and maintain the stationary position by controlling a movement of the unmanned aircraft 16. When the unmanned aircraft 16 is in the stationary position, the controller 18 may trigger X-ray image acquisition to acquire an X-ray image of at least a part of the patient 15 arranged between the X-ray source 12 and the X-ray detector 14. The stationary position may be a stationary flight position or a stationary position on ground.

Summarizing, positioning of the unmanned aircraft 16 with respect to the patient 15 could be made by selecting the imaging task e.g. via an application on the control device 102 and by automatically adjusting the current source to detector distance and/or at least one value of any other acquisition parameter. Further, positioning may be controlled externally via the control device 102 or it may be performed automatically by the imaging system 10 based on image recognition. For example, a person could point to the area of interest on the control device 102 and the unmanned aircraft 16 may navigate itself to the location. Alternatively, a command could be issued for a set of imaging tasks, such as e.g. lower leg, knee, arm, head, thorax, or any other body part. Such command could be an integral part of the request for delivery.

Moreover, the controller 18 may be configured to determine a current relative orientation of the X-ray source 12 and the X-ray detector 14 based on processing image data of the camera 22. Further, the controller 18 may be configured to align the X-ray source 12 and the X-ray detector with respect to each other based on image recognition, based on processing image data of images captured with the camera 22, and/or based on the determined current relative orientation. For instance, the controller 18 may determine, based on image processing of images captured with the camera 22, a detector plane in the image data, wherein the controller 18 may determine that the X-ray source 12 and the X-ray detector 14 are aligned when the detector plane comprises parallel detector edges. Accordingly, the controller 18 may navigate and/or maneuver the unmanned aircraft 18, until the X-ray source 12 and the X-ray detector 14 are aligned.

Apart from that, the imaging system 10 may comprise a microphone and/or a speaker to communicate with the patient 15. By means of the microphone and/or speaker, instructions on how to position himself may be provided to the patient 15. Such instructions may e.g. comprise "move your leg to the left" or "lift your left leg". This may allow to ideally position the patient 15 with respect to the X-ray source 12, the X-ray detector 14 and/or the unmanned aircraft 16.

Moreover, based on processing image data of images captured with the camera 22, any movement of the patient 15 during X-ray image acquisition may be compensated by means of the controller 18. For instance, based on image recognition, the controller 18 may determine a profile and/or an edge of the patient 15 in one of the images, and the controller 18 may determine a shift of the profile and/or edge in consecutive images. Based on this shift, the movement of the patient 15 may be corrected for.

Moreover, for stabilization of the unmanned aircraft 16, particularly during X-ray image acquisition, the controller 18 may process and/or evaluate one or more sensor signals of one or more sensors of the unmanned aircraft 16. The unmanned aircraft 16 may comprise at least one of an accelerometer, a distance sensor, an optical sensor, a visual sensor, a radar distance sensor, a laser distance sensor, a gyroscope, a speed sensor, a wind speed sensor and/or any other type of sensor. Sensor signals of any of those sensors may be used to stabilize the unmanned aircraft 16 during X-ray image acquisition.

Alternatively or additionally, the controller 18 may process the sensor signals for image reconstruction of the X-ray image and/or for compensating for any movement of the patient 15 and/or any relative movement of the X-ray source 12 with respect to the X-ray detector 14 during X-ray image acquisition.

Further, the controller 18 may be configured to transmit data related to the X-ray image via the communication interface 20 e.g. to the control device 102 and/or to a control station. The data related to the X-ray image may comprise X-ray image data and/or the actual X-ray image. Moreover, the data related to the X-ray image may comprise the values of the acquisition parameters as determined by the controller 18 based on the imaging task. Further, the controller 18 may be configured to transmit data related to sensor signals of any further sensor of the unmanned aircraft 16. These data maybe used e.g. for image reconstruction at the control device 102 and/or at the control station.

Moreover, the controller 18 may be configured to determine whether any further object, particularly a further human, may be present in the field of view of the X-ray source 12 based on processing image data of images captured with the camera 22. Accordingly, the controller 18 may be configured to trigger X-ray image acquisition, if it detects that no further human is present in the field of view of the X-ray source 12. Accordingly, an X-ray trigger may be interlocked by the controller 18 until the controller 18 ensured safety, i.e. ensured that no human other than the patient 15 is in the field of view. This may ensure safety during X-ray image acquisition.

Apart from that, the X-ray detector 14 may be equipped with self-triggering capability to avoid any synchronization issues and/or to ensure that the X-ray source 12 and the X-ray detector are synchronized.

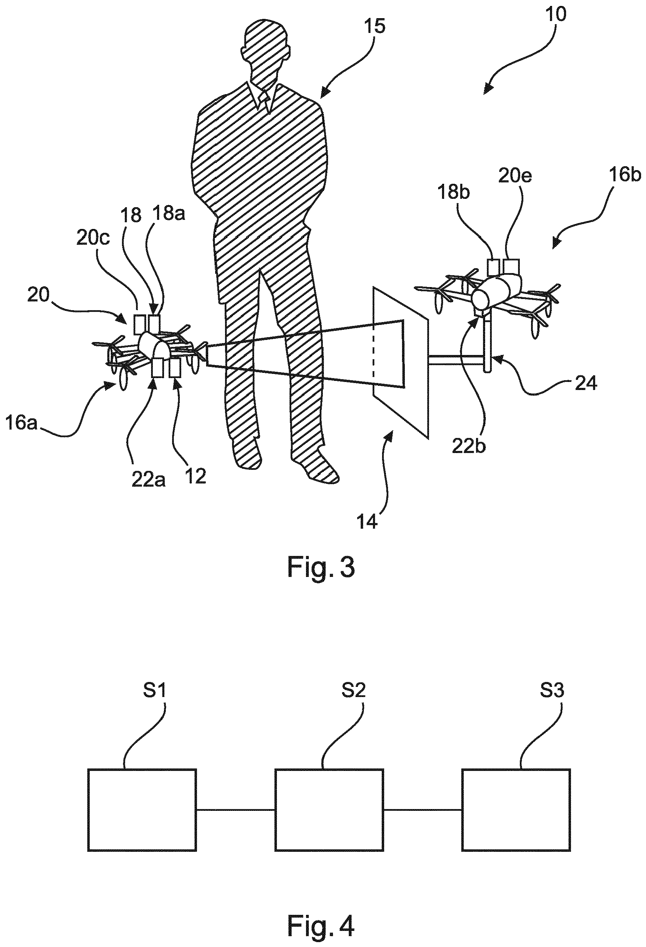

Fig. 3 shows schematically an X-ray imaging system 10 according to a further exemplary embodiment. If not stated otherwise, the imaging system 10 of Fig. 3 comprises the same elements, features and/or functions as the imaging system 10 described with reference to Figs. 1 and 2.

Compared to the embodiment described with reference to Fig. 2 the imaging system 10 of Fig. 3 comprises an unmanned aircraft 16a, on which the X-ray source 12 is arranged, and a further unmanned aircraft 16b, on which the X-ray detector 14 is arranged.

The unmanned aircraft 16a comprises a camera 22a for capturing images of a surrounding of the unmanned aircraft 16a. The further unmanned aircraft 16b comprises a further camera 22b for capturing images of a surrounding of the further unmanned aircraft 16b.

The communication interface 20 comprises a communication element 20c arranged on and/or comprised in the unmanned aircraft 16a and a further communication element 20e arranged on and/or comprised in the further unmanned aircraft 16b. By means of the communication elements 20c, 20e any data, information and/or signals may be exchanged between the unmanned aircraft 16a and the further unmanned aircraft 16b. Further, the X-ray source 12 may comprise a communication element 20a, the X-ray detector 14 may comprise a communication element 20b, and/or the controller may comprise a communication element 20d.

The unmanned aircraft 16a comprises a sub-controller 18a of the controller 18 and the further unmanned aircraft 16b comprises a further sub controller 18b of the controller 18. Alternatively, the controller 18 maybe entirely integrated into the unmanned aircraft 16a or the further unmanned aircraft 16b. The further unmanned aircraft 16b may be controlled by the controller 18 via the communication interface 20 and/or the communication elements 20c, 20e.

For instance, the unmanned aircraft 16a may be a master aircraft 16a, and/or the controller 18 and/or sub-controller 18a may provide navigation signals to the further unmanned aircraft 16b via the communication interface 20. The further unmanned aircraft 16b may be a slave aircraft and/or may move according to the navigation signals.

Further, each of the unmanned aircrafts 16a, 16b may comprise one or more sensors, such as e.g. an accelerometer, a distance sensor, an optical sensor, a visual sensor, a radar distance sensor, a laser distance sensor, a gyroscope, a speed sensor, a wind speed sensor and/or any other type of sensor. Via the communication elements 20c, 20d data related to sensor signals of any of those sensors maybe exchanged between the unmanned aircrafts 16a, 16b. These data may be processed by the controller 18, by the sub-controller 18a and/or by the sub-controller 18b. This may allow to coordinate a movement of both the unmanned aircraft 16a and the further unmanned aircraft 16b.

The unmanned aircraft 16a may, e.g. based on a control signal received from the control device 102, transport the X-ray source 12 to the imaging site, and the further unmanned aircraft 16b may transport the X-ray detector 14 to the imaging site.

In the embodiment depicted in Figure 3, the further unmanned aircraft 16b comprises an extendable support 24, wherein one end of the support 24 is affixed to the further unmanned aircraft 16b and a further end of the support 24 is affixed to the X-ray detector 14. Alternatively or additionally, the unmanned aircraft 16a may comprise an extendable support 24, on which the X-ray source 12 may be affixed to.

For positioning and/or aligning the X-ray source 12 and the X-ray detector 14 with respect to each other, the controller 18 is configured to actuate the extendable support 24, such that the X-ray detector 14 and the X-ray source 12 are arranged opposite to each other and/or such that they are aligned with respect to each other.

As described with reference to Figure 2, the imaging task may be transmitted to and/or selected by the controller 18. For instance, the patient 15 could point to a region of interest, wherein the controller 18 may be configured to determine the region of interest based on image recognition using at least one of the cameras 22a, 22b and/or based on image processing image data of the images captured with at least one of the cameras 22a, 22b. Alternatively or additionally, the imaging task may e.g. be selected on the control device 102 via an application, and a control signal containing data indicative of and/or related to the imaging task may be transmitted to the imaging system 10 via the communication interface 20.

Further, as described with reference to Figure 2, the controller 18 is configured to determine a value of at least one acquisition parameter based on the imaging task, wherein the at least one acquisition parameter is selected from the group consisting of a source to detector distance, an X-ray voltage, an X-ray current, a field of view, a magnification, and an acquisition time. Based on the imaging task, the controller 18 may calculate one or more values of one or more acquisition parameters and/or the values of the acquisition parameters may be stored in a storage device and/or a data storage of the imaging system 10, and the controller 18 may select the values based on the imaging task.

Further, the controller 18 is configured to automatically adjust the value of the at least one acquisition parameter, particularly the value of every acquisition parameter, by controlling a movement of the unmanned aircraft 16a, a movement of the further unmanned aircraft, the unmanned aircraft 16a, the further unmanned aircraft 16b, the X-ray source 12 and/or the X-ray detector 14. Specifically, the X-ray voltage, the X-ray current, the field of view, and/or the magnification may automatically be adjusted by controlling the X-ray source 12, the X-ray detector 14, the unmanned aircraft 16a and the further unmanned aircraft 16b with the controller 18 and/or with any of the sub-controllers 18a, 18b.

By way of example, the controller 18 and/or any of sub-controllers 18a, 18b may determine a source to detector distance based on the imaging task. Based on processing image data of any of the cameras 22a, 22b, the controller 18 and/or any of sub-controllers 18a, 18b determines a current and/or actual source to detector distance, and compares this current source to detector distance with the source to detector distance as determined based on the imaging task. Further, the controller 18 and/or any of sub-controllers 18a, 18b may maneuver and/or position at least one of the unmanned aircraft 16a and the further unmanned aircraft 16b such that the current source to detector distance matches the source to detector distance as determined based on the imaging task.

The controller 18 and/or any of sub-controllers 18a, 18b may also be configured to determine a stationary position of the unmanned aircraft 16a with respect to the patient 15, which stationary position may be suitable for the imaging task. Also, the controller 18 and/or any of sub-controllers 18a, 18b may determine a further stationary position of the further unmanned aircraft 16b, which further stationary position may be suitable for the imaging task. The stationary position and/or the further stationary position may be a stationary flight position or a stationary position on ground, respectively.

Based on image recognition and/or based on processing image data of one or more images captured with at least one of the cameras 22a, 22b, the controller and/or any of sub-controllers 18a, 18b may control a relative movement of the unmanned aircraft 16a and the further unmanned aircraft 16b, wherein any of the unmanned aircrafts 16a, 16b may be moved three-dimensionally. For instance, the controller and/or any of sub-controllers 18a, 18b may detect and/or track the further unmanned aircraft 16b in the image data of the images captured with the camera 22a. The controller 18 and/or any of sub-controllers 18a, 18b may maneuver the unmanned aircraft 16a and/or the further unmanned aircraft to control a position of the X-ray source 12 with respect to the patient 15, a position of the X-ray detector 14 with respect to the patient 15, a relative orientation of the X-ray source 12 and the X-ray detector 14, and/or a current source to detector distance between the X-ray source 12 and the X-ray detector 14.

A position, e.g. the stationary position, of the unmanned aircraft 16a and/or a position, e.g. the further stationary position, of the further unmanned aircraft 16b may be locked by the controller 18 and/or the positions of the unmanned aircrafts 16a, 16b may be maintained during X-ray image acquisition. This way, the current source to detector distance may be controlled and/or the current source to detector distance matching the one determined based the imaging task may be maintained. Alternatively or additionally, the controller 18 and/or any of sub-controllers 18a, 18b may be configured to determine a current relative orientation of the X-ray source 12 and the X-ray detector 14 based on processing image data of at least one of the cameras 22a, 22b. Further, the controller 18 and/or any of sub-controllers 18a, 18b maybe configured to align the X-ray source 12 and the X-ray detector 14 with respect to each other based on image recognition and/or based on the determined current relative orientation, as described in detail with reference to Figure 2.

Summarizing, the alignment of the X-ray source 12 and the X-ray detector 14 and/or the alignment of the unmanned aircraft 16a and the further unmanned aircraft 16b may be done and/or accomplished by image recognition and/or locking the position of one device with respect to the other one. For example, the X-ray source 12 and/or the unmanned aircraft 16a may "see", by means of the camera 22a, the X-ray detector 14 and/or the further unmanned aircraft 16b, and can maneuver itself and/or the further unmanned aircraft 16b to control the position and the current source to detector distance. This can be done visually and/or by processing image data of images captured with at least one of the cameras 22a, 22b, by knowing the size of the detector, i.e. the reference detector size, and the reference image size of the cameras 22a, 22b, based on which the current source to detector distance may be computed.

It is noted, however, that also only a relative position of the unmanned aircraft 16a with respect to the further unmanned aircraft 16b may be locked and/or maintained.

By way of example, the controller 18 and/or any of sub-controllers 18a, 18b may maneuver the unmanned aircraft 16a and/or the further unmanned aircraft 16b along a trajectory around the patient 15 by controlling a relative movement of the unmanned aircraft 16a with respect to the further unmanned aircraft 16b. While moving the unmanned aircrafts 16a, 16b and thereby while moving the X-ray source 12 and detector 14 a plurality of X-ray images may be acquired and/or stored e.g. in a data storage. Accordingly, a part of the patient 15 maybe scanned. This enables Tomo-Synthesis applications and/or allows to move the source 12 and detector 14 in a C-arm like trajectory around the patient 15 without any mechanical constraints.

Data related to the trajectory may be stored in the data storage and/or may be transmitted via communication interface 20 to the control device 102 and/or a control station. Alternatively or additionally, sensor data related to sensor signals of any of the sensors of the unmanned aircrafts 16a, 16b may be stored in the data storage and/or may be transmitted via communication interface 20 to the control device 102 and/or the control station. Further, data related to the X-ray image may be transmitted. The controller 18 and/or the control device 102 may then process the X-ray image data taking into account any of the data related to the trajectory and/or any of the sensor data. This may allow to reconstruct and/or render a high-quality X-ray image and/or an X-ray scan. Also, motion artefacts may be corrected for based on the trajectory data and/or the sensor data.

Briefly summarized, the unmanned aircrafts 16a, 16b may not only be used to deploy the X-ray source 12 and detector 14 to a remote location, but also to position the source 12 and the detector 14 for the imaging task. That is, both unmanned aircrafts 16a, 16b may ensure the right imaging position for a given protocol and/or imaging task, align themselves and keep the necessary source to detector distance, e.g. while in stationary position and/or stationary flight position. To this effect the unmanned aircrafts 16a, 16b may be equipped with visual recognition systems and/or cameras 22a, 22b, not only for positioning and alignment tasks, but also to ensure X-ray safety. For instance, both unmanned aircrafts 16a, 16b may maintain a stationary position around the area of the patient 15 that requires an X-ray image. This way, the X-ray acquisition and positioning may not require any mechanical support for the acquisition itself. E.g. depending on the severity of the emergency, the patient 15 maybe imaged in different positions, such as e.g. a standing position. The patient 15 may also however be sitting or lying on a table or bed, where the use of the unmanned aircrafts 16a, 16b may still be possible. Further, the patient 15 may be instructed by a microphone and/or speaker arranged on at least one of the unmanned aircrafts 16a, 16b, as described with reference to Figure 2.

Moreover, any of the unmanned aircrafts 16a, 16b may be equipped with a collision avoidance system, allowing to get much closer to the patient 15 or surrounding elements without any risk of injuries or material damage. This may make it possible not only to carry out their task with the patient 15 in different positions, but also to operate indoors. Since the unmanned aircrafts 16a, 16b may be kept reasonably close to the patient 15, high power X-ray sources 12 may not be required. Accordingly, the X-ray source 12 and/or the X-ray detector 14 may be lightweight and/or battery-powered. Further, each of the unmanned aircrafts 16a, 16b may be configured to carry well in excess of 2 kg.

Further, the controller 18 may be configured to control and/or adjust a magnification of the X-ray image based on adjusting a distance between the unmanned aircraft 16a and/or the X-ray source 12 and the patient 15. Alternatively or additionally, a distance between the further unmanned aircraft 16b and/or the X-ray detector 14 and the patient 15 maybe adjusted.

Moreover, it is noted that in Figures 2 and 3 a projection is parallel to ground and/or a detector plane of the detector 14 is perpendicular to ground. However, it is to be noted that the detector plane of the detector 14 may be arranged at an arbitrary angle with respect to ground, such as e.g. in a 90° vertical orientation. For instance, the patient 15 may be laying on a table or a stretcher, wherein the X-ray source 12 may be arranged underneath the table pointing upwards and the X-ray detector 14 may overfly above and/or maybe arranged above the patient 15 and/or table. Vice, versa also the X-ray detector 14 may arranged underneath the table and the X-ray source 12 maybe arranged above the patient 15 and/or table. Further, the extendable support 24 may comprising a rotating axle and/or mechanical joint for this purpose.

Fig. 4 shows a flow chart illustrating steps of a method for acquiring an X-ray image of an object 15 with an X-ray imaging system 10 according to an embodiment. The X-ray imaging system 10 may refer to an imaging system 10 as described with reference to any of the previous figures and/or the X-ray imaging arrangement 100 as described with reference to Figure 1.

Particularly, the X-ray imaging system 10 comprises an X-ray source 12, an X-ray detector 14 and at least one unmanned aircraft 16, wherein the X-ray source 12 or the X-ray detector 14 is mounted to the unmanned aircraft 16.

In a step S1 the at least one unmanned aircraft 16 is positioned with respect to an object 15 to be examined, such that the object 15 is arranged between the X-ray source 12 and the X-ray detector 14. Further, in step S1 a control signal maybe received by the imaging system 10, e.g. from a control device 102, as described in detail in Figures 2 and 3. Also, one or more values of one or more further acquisition parameters may be determined in step S1, as described with reference to previous figures.

In a further step S2, the X-ray source 12 and the X-ray detector 14 are aligned by maneuvering and/or positioning the at least one unmanned aircraft 16. In step S2, a current source to detector distance may be determined based on processing image data of images captured with a camera 22 of the unmanned aircraft 16. The current source to detector distance may be compared with a source to detector distance determined based on an imaging task, and the unmanned aircraft 16 may be maneuvered such that the current source to detector distance matches the source to detector distance as determined based on the imaging task, as described in detail with reference to Figures 2 and 3.

In a further step S3, an X-ray image of the object 15 arranged between the X-ray source 12 and the X-ray detector 14 is captured and/or acquired.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An X-ray imaging system (10) for medical imaging, the imaging system (10) comprising:
an X-ray source (12) for emitting X-rays;
an X-ray detector (14) for detecting X-rays;
at least one unmanned aircraft (16); and
at least one controller (18) for controlling at least one of the X-ray source (12), the X-ray detector (14), and the at least one unmanned aircraft (16);
wherein the X-ray source (12) or the X-ray detector (14) is arranged on the at least one unmanned aircraft (16); and
wherein the X-ray imaging system (10) is configured to capture an X-ray image of an object (15) arranged between the X-ray source (12) and the X-ray detector (14).

2. The X-ray imaging system (10) according to claim 1,
wherein the X-ray imaging system (10) is configured to capture an X-ray image of the object (15) arranged between the X-ray source (12) and the X-ray detector (14), when the at least one unmanned aircraft (16) is in a stationary position; and
wherein the stationary position of the unmanned aircraft (16) is a stationary flight position or a stationary position on ground.

3. The X-ray imaging system (10) according to any of the preceding claims,
wherein the X-ray imaging system (10) comprises a further unmanned aircraft (16b);
wherein the X-ray source (12) is arranged on the unmanned aircraft (16a) and the X-ray detector (14) is arranged on the further unmanned aircraft (16b); and
wherein the imaging system (10) is configured to capture the X-ray image of the object (15), when the unmanned aircraft (16a) is in the stationary position and the further unmanned aircraft (16b) is in a further stationary position.

4. The X-ray imaging system (10) according to any of the preceding claims,
wherein the at least one unmanned aircraft (16) comprises an extendable support (24), on which the X-ray source (12) or the X-ray detector (14) is arranged; and
wherein the controller (18) is configured to position the X-ray source (12) or the X-ray detector (14) by actuating the extendable support (24) of the unmanned aircraft (16), such that the X-ray source (12) and the X-ray detector (14) are arranged opposite to each other.

5. The X-ray imaging system (10) according to any of the preceding claims,
wherein the at least one controller (18) is configured to determine a value of at least one acquisition parameter based on an imaging task, the imaging task specifying at least a part of the object (15) to be examined with the X-ray imaging system (10); and
wherein the at least one controller (18) is configured to automatically adjust the value of the at least one acquisition parameter by controlling at least one of the X-ray source (12), the X-ray detector (14) and the at least one unmanned aircraft (16).

6. The X-ray imaging system (10) according to claim 5,
wherein the at least one acquisition parameter is selected from the group consisting of a source to detector distance, an X-ray voltage, an X-ray current, a field of view, a magnification, and an acquisition time.

7. The X-ray imaging system (10) according to any of the preceding claims, further comprising:
at least one communication interface (20);
wherein the X-ray imaging system (10) is configured to receive data related to an imaging task via the communication interface (20), the imaging task specifying at least a part of the object (15) to be examined with the X-ray imaging system (10); and/or
wherein the X-ray imaging system (10) is configured to transmit data related to the X-ray image via the communication interface (20).

8. The X-ray imaging system (10) according to any of the preceding claims,
wherein the at least one unmanned aircraft (16) comprises at least one camera (22);
wherein the controller (18) is configured to position the at least one unmanned aircraft (16) with respect to the object (16) based on image recognition using the at least one camera (22), such that at least a part of the object (15) to be examined with the X-ray imaging system (10) is arranged between the X-ray source (12) and the X-ray detector (14).

9. The X-ray imaging system (10) according to any of the preceding claims,
wherein the imaging system (10) comprises a further unmanned aircraft (16b);
wherein the X-ray source (12) is arranged on the unmanned aircraft (16a) and the X-ray detector (14) is arranged on the further unmanned aircraft (16b);
wherein the unmanned aircraft (16a) and the further unmanned aircraft (16b) comprise at least one camera (22a, 22b);
wherein the at least one controller (18) is configured to align the X-ray source (12) with respect to the X-ray detector (14) by positioning the unmanned aircraft (16a) and the further unmanned aircraft (16b) with respect to each other based on image recognition using the at least one camera (22a, 22b).

10. The X-ray imaging system (10) according to claim 9,
wherein the at least one controller (18) is configured to determine a current relative orientation of the X-ray source (12) and the X-ray detector (14) based on image data of the at least one camera (22a, 22b); and/or
wherein the at least one controller (18) is configured to determine a current source to detector distance between the X-ray source (12) and the X-ray detector (14) based on image data of the camera (22a, 22b), based on a reference size of the X-ray detector (14), and based on a reference image size of the at least one camera (22a, 22b).

11. The X-ray imaging system (10) according to claim 10,
wherein the controller (18) is configured to maintain the current relative orientation and/or the current source to detector distance during X-ray image acquisition by maneuvering at least one of the unmanned aircraft (16a) and the further unmanned aircraft (16b) and by positioning the unmanned aircraft (16a) and the further unmanned aircraft (16b) with respect to each other.

12. The X-ray imaging system (10) according to any of claims 9 to 11,
wherein the controller is configured to maneuver the unmanned aircraft (16a) and/or the further unmanned aircraft (16b) along a trajectory around the object (15); and
wherein the imaging system (10) is configured to scan at least a part of the object (15) based on capturing a plurality of X-ray images along the trajectory.

13. The X-ray imaging system (10) according to any of the preceding claims,
wherein the at least one unmanned aircraft (16) comprises at least one camera (22) configured to capture a plurality of images during X-ray image acquisition of the object (15);
wherein the controller (18) is configured to determine a movement of the object (15) during X-ray image acquisition based on image data of the plurality of images captured with the at least one camera (22); and
wherein the controller (18) is configured to compensate and/or correct for the movement of the object (15) during X-ray image acquisition based on image data of the plurality of images captured with the at least one camera (22) during X-ray image acquisition.

14. An X-ray imaging arrangement (100) for medical X-ray imaging, comprising:
an X-ray imaging system (10) according to any of the preceding claims; and
a control device (102) for at least partly controlling the X-ray imaging system (10) based on remote control.

15. A method for acquiring an X-ray image of an object (15) with an X-ray imaging system (10) comprising an X-ray source (12), an X-ray detector (14) and at least one unmanned aircraft (16), wherein the X-ray source (12) or the X-ray detector (14) is mounted to the unmanned aircraft (16), the method comprising the steps of:
positioning the at least one unmanned aircraft (16) with respect to an object (15) to be examined, such that the object (15) is arranged between the X-ray source (12) and the X-ray detector (14);
aligning the X-ray source (12) and the X-ray detector (14) by maneuvering and/or positioning the at least one unmanned aircraft (16); and
capturing an X-ray image of the object (15) arranged between the X-ray source (12) and the X-ray detector (14).
